# EUROPEAN PATENT APPLICATION

(11) **EP 0 995 733 A1**
(43) Date of publication of application: **26.04.2000**
(21) Application number: 99203452.0
(22) Date of filing: 21.10.1999
(51) Int. Cl.: C07C 17/12, C07C 25/22

(54) **Improved process for the preparation of TMPI derivatives**

(30) Priority: 22.10.1998 IL 12669598
(71) Applicant: Bromine Compounds Ltd., Beer-Sheva 84101 (IL)
(72) Inventor: Kornberg, Nurit, Lehavim (IL); Fishler, Theodor Morel, Haifa (IL); Antebi, Salomone, Haifa (IL)
(74) Representative: Long, Giorgio

(57) **Abstract**

A process for the preparation of polybrominated TMPI compounds, comprising reacting TMPI in the presence of a catalyst, in a solvent selected from the group consisting essentially of liquid bromine, dihaloalkane, such as dichloroethane, or a mixture of two or more dihaloalkanes. The mixture of dihaloalkanes may comprise one or more of dichloromethane, chlorobromomethane and dibromomethane. The temperature during the addition of TMPI may vary between about 0°C and 40°C. When the catalyst is selected to be AlCl₃, the temperature during the addition of TMPI may vary between about 0°C and 5°C.

## Description

### Field of the Invention

The present invention relates to an improved process for the preparation of polybrominated compounds applied in compositions as additive fire retardants, said additive fire retardants being halogenated derivatives of TMPI (1,1,3-trimethyl-3-phenyl indan).

### Background of the Invention

Halogenated fire retardants are a very important family of plastic additives, a number of these being halogenated compounds. The growing importance of halogenated flame retardants is due to their efficiency and thermal stability, even under severe conditions, as well as their strong contribution to reduction of smoke toxicity under fire (*V. Babrauskas et al. - NBS Special Publication 749 - U.S. Government Printing Office, Washington (1988))*.

The preparation of TMPI is disclosed in DE 2,906,294, DE 2,659,597 and USP 3,161,692. MPI (1-methyl-3-phenyl indan) can be obtained by condensing two molecules of styrene (*Kirk-Othmer Encyclopedia of Chemical Technology*, Vol. 3, p. 705). Petropoulos and Fisher in *J. Am. Chem. Soc.* 80 1938 (1958), relate to the dimerization of alpha-methylstyrene and/or ring alkylated alpha methylstyrene to yield TMPI compounds.

Additive fire retardants, the process for their preparation and polymeric compositions containing them are described in USP 5,679,736.

EP 138766 gives an example of the dimerization of 4-chloro-alpha-methylstyrene to produce 6-chloro-1,3,3-trimethyl-1-(4-chlorophenyl)indan.

USP 5,710,354 teaches a two-step process for the preparation of polybrominated indanes, which requires the step of forming an intermediate to be further brominated. This is a complex process, requiring close control of the intermediate formation.

It is a purpose of the present invention to provide an improved process for the preparation of polybrominated TMPI. Such a process provides substantial industrial advantages, as will be fully appreciated from the description to follow.

Other objects and advantages of the invention will become apparent as the description proceeds.

### Summary of the Invention

The process for the preparation of polybrominated TMPI compounds according to the invention comprises reacting TMPI in the presence of a catalyst and in a solvent selected from the group consisting essentially of liquid bromine, haloalkane, or a mixture of two or more dihaloalkanes.

According to a preferred embodiment of the invention the dihaloalkane is a dichloroalkane. Still more preferably the dichloroalkane is dichloroethane. According to another preferred embodiment of the invention the mixture of dihaloalkanes comprises two or more of dichloromethane (DCM), chlorobromomethane (CBM) and dibromomethane (DBM).

The term "polybrominated", as used herein, is meant to indicate compounds substituted by three or more bromine atoms.

According to a preferred embodiment of the invention a solution of TMPI is added while keeping the temperature during such addition between about 0°C and about 40°C. According to another preferred embodiment of the invention the reaction temperature after the TMPI addition is kept between about 30°C and about 75°C.

Any suitable catalyst can be employed in the reaction of the invention. According to a preferred embodiment, however, the catalyst is chosen from among Fe powder, FeCl₃, AlCl₃, SbCl₃, and is used in an amount of at least 0.4% by weight, 12-14% molar vs TMPI in the case of AlCl₃.

The fire retardant compounds obtained by the process of the invention are added to polymers and exhibit substantially improved thermal stability.

All above and other characteristics and advantages of the invention will be better understood through the following illustrative and non-limitative description of preferred embodiments thereof.

### Example 1

Bromine (522 g, 3.3 mole) and antimony trichloride (5.9 g, 0.026 mole) were placed into a 1 liter reactor, equipped with a mechanical stirrer, and were dissolved in a dry solvent mixture of 55% DBM, 35% CBM, 10% DCM, total of 640 g.

The mixture was cooled to 4°C and a 50% solution of TMPI (80 g, 0.34 mole) in the same dry solvent mixture was added over a period of 2 hours.

The temperature was kept at 10°C to the end of the addition, the reaction mixture was heated to 30°C for 1.5 hours until HBr emission subsided, and chlorine (132 g, 1.86 mole) was slowly added via a dip-tube.

The solid was separated to give 189.5 g product. It was composed of a mixture of 93% octabromo-TMPI and 7% octabromo-chloro-TMPI

### Example 2

A dry solvent mixture (1378 g, 689 ml) (DBM: CBM: DCM = 55:35:10, respectively) and AlCl₃ (13.3 g, 0.1 mole) and bromine (1453 g, 9.1 mole) were placed into a 2 liter jacket reactor at 0°C. A 50%, TMPI solution (472.4 g, 1 mole) was added dropwise over 5 hours, and the temperature was kept at 2-3°C. At the end of the addition, the temperature was raised to 30°C during 1 hour, and maintained for two hours, the mixture was heated to 40°C for 30 minutes.

The solid was filtered in a 90% yield. The product composition was 90% OctabromoTMPI.

### Example 3

Bromine (300 ml, 930 g, 5.8 mole) and Fe powder (4 g, 0.07 mole) were placed into a 500 ml jacketed reactor equipped with an efficient stirrer. TMPI (85 g, 0.36 mole) was added by peristaltic pump. at 40°C for two hours, after which, the mixture was heated to 57°C for one hour.

400 ml of water were added to the mixture to dissolve the bulk of FeBr₃. The mixture was heated to vaporize the excess bromine.

The product contained 96% Octabromo-TMPI.

### Example 4

Bromine (173 g, 1.08 mol) and AlCl₃ (3.85 g, 0.0289 mol) in dichloroethane (100 ml) were placed into a three-necked 250 ml flask and a solution of TMPI (23.6 g : 0.1 mol) in 120 gr dichloroethane was added dropwise at 40°C during 190 minutes. The temperature was increased gradually to 75°C. The reaction mixture was maintained for 30 min at 75°C, then cooled to 20°C. The excess bromine was neutralized with sodium bisulfite and the solid was filtered and washed with water. The dry solid was obtained in 72% yield.

### Example 5

Bromine, 950 g (5.94 mole), FeCl₃, 2.7 g (0.017 mole) were placed in a 11 reactor equipped with a mechanical stirrer, reflux condenser thermometer and addition funnel. The mixture was kept at 20°C during the addition of melted TMPI (∼60°C), 85 g (0.36 mole) by gentle cooling. Two hours after starting the addition, the mixture was heated gradually to 50-55°C and was kept at this temperature for another two hours. Water, 400 ml, was added gradually, while distilling excess bromine. After neutralizing the residual bromine with sodium bisulfite solution, the product was further washed with 2x250 ml water and filtered. The product was obtained in 90% yield and contained 95% Octabromo-TMPI.

### Example 6

Bromine, 322 g (2.01 mole) and antimony chloride, SbCl₃, 28.8 g(0.127 mole) were placed into a 11 reactor equipped with a mechanical stirrer, reflux condenser thermometer and addition funnel, and were dissolved in 250 g dry solvent mixture of 55%w Dibromomethane, DBM, 35% Chlorobromomethane, CBM, and 10% Dichloromethane, DCM. A 50% solution of TMPI in the same solvent mixture, 100 g (0.21 mole) was added dropwise over 30 minutes at 20°C. The reaction mixture was heated to 70°C for another 2 hours. After cooling to ambient temperature, water and sodium bisulfite solution were added. After two additional washings with 2x250 ml of water, the organic layer was evaporated to constant weight. A highly viscous product, 105 g, 97% yield, with 54% bromine content was obtained. The calculated average composition of this product was 50% by weight of TribromoTMPI and 50% TetrabromoTMPI.

### Example 7

Bromine, 326 g (2.04 mole) and zinc chloride, ZnCl₂, 17.8 g(0.13 mole) were placed into a 11 reactor equipped with a mechanical stirrer, reflux condenser thermometer and addition funnel, and were dissolved in 250 g dry solvent mixture of 55%w Dibromomethane, DBM, 35% Chlorobromomethane, CBM, and 10% Dichloromethane, DCM. A 50% solution of TMPI in the same solvent mixture, 100 g (0.21 mole) was added dropwise over 30 minutes at 20°C. The reaction mixture was heated to 70°C for another 4.5 hours. After cooling to ambient temperature, water and sodium bisulfite solution were added. After two additional washings with 2x250 ml of water, the organic layer was evaporated to constant weight. A highly viscous product, 103 g, 97% yield, with 51.8% bromine content, was obtained. The calculated average composition of this product was 85% by weight of Tribromo-TMPI and 15% Tetrabromo-TMPI.

### Example 8

Bromine, 500 ml (9.7 mole) and SnCl₂, 14.2 g(0.075 mole) were placed into a 11 reactor equipped with a mechanical stirrer, reflux condenser thermometer and addition funnel. The mixture was kept at 15°C during the addition of melted TMPI (∼60°C), 77 g (0.33 mole) by gentle cooling. The reaction mixture was kept for 2 hours after addition at 20°C, and afterwards the mixture was heated gradually to 50-55°C and was kept at this temperature for another 4 hours. Water, 400 ml, was added gradually, while distilling excess bromine. After neutralizing the residual bromine with sodium bisulfite solution, the product was further washed with 2x250 ml water and filtered. The product contained, according to HPLC, 75% Area TribromoTMPI and 11% TetrabromoTMPI.

## Claims

1. A process for the preparation of polybrominated TMPI compounds, comprising reacting TMPI in the presence of a catalyst, in a solvent selected from the group consisting essentially of liquid bromine, haloalkane, or a mixture of two or more dihaloalkanes.

2. A process according to claim 1, wherein the dihaloalkane is dichloroethane.

3. A process according to claim 1, wherein the mixture of dihaloalkanes comprises one or more of dichloromethane, chlorobromomethane and dibromomethane.

4. A process according to any one of claims 1 to 3, wherein the temperature during the addition of TMPI is comprised between about 0°C and 40°C.

5. A process according to claim 1, wherein the catalyst is AlCl₃, and the temperature during the addition of TMPI is comprised between about 0°C and 5°C.

6. A process according to claim 4, wherein the catalyst is chosen from among Fe, FeCl₃, SbCl₃, ZnCl₂ and SnCl₂, and the temperature during the addition of TMPI is comprised between about 0°C and 40°C.

7. A process according to any one of claims 1 to 6, wherein the reaction temperature after TMPI addition is comprised between about 20°C to 75°C.

8. A process according to any one of claims 1 to 7, wherein the catalyst is chosen from among Fe, FeCl₃, AlCl₃, SbCl₃, ZnCl₂ and SnCl₂.

9. A process according to claim 8, wherein the catalyst is used in amounts of at least 0.4% by weight.

10. A process according to claim 9, wherein the catalyst is AlCl₃ and is present in an amount of about 12% to 14% by mole, relative to TMPI.

11. A process according to any one of claims 1 to 9, wherein the polybrominated TMPI is octabromo-1,1,3-trimethyl-3-phenyl indan.

12. Polybrominated TMPI, whenever prepared by the process of any one of claims 1 to 11.

13. Octabromo-1,1,3-trimethyl-3-phenyl indan, whenever prepared by the process of any one of claims 1 to 11.
